# EUROPEAN PATENT APPLICATION

(11) **EP 4 144 360 A1**
(43) Date of publication of application: **08.03.2023**
(21) Application number: 20933484.6
(22) Date of filing: 03.07.2020
(51) Int. Cl.: A61K 38/17, A61K 9/19, A61P 31/14

(54) **SARS-COV-2 ANTIVIRAL DRUG ANTIPROVIR**

(30) Priority: 30.04.2020 RU 2020116573
(71) Applicant: Ivashchenko, Andrey Alexandrovich, Moscow 127576 (RU); Ivachtchenko, Alexandre Vasilievich, Hallandale, FL 33009 (US); Savchuk, Nikolay Filippovich, Rancho Santa Fe, CA 92067 (US); Ivachtchenko, Alena Alexandrovna, Hallandale, Florida 33009 (US); Loginov, Vladimir Grigorievich, Moscow, 121248 (RU); Topr, Mikhail, New York 10023 (US)
(72) Inventor: Ivashchenko, Andrey Alexandrovich, Moscow 127576 (RU); Ivachtchenko, Alexandre Vasilievich, Hallandale, FL 33009 (US); Savchuk, Nikolay Filippovich, Rancho Santa Fe, CA 92067 (US); Ivachtchenko, Alena Alexandrovna, Hallandale, Florida 33009 (US); Loginov, Vladimir Grigorievich, Moscow, 121248 (RU); Topr, Mikhail, New York 10023 (US)
(74) Representative: Zellentin & Partner mbB Patentanwälte
(86) International application number: PCT/RU2020/000328
(87) International publication number: WO 2021/221532

(57) **Abstract**

This invention relates to a novel anti-SARS-CoV-2 virus agent Antiprovir to be used for the prevention and treatment of coronavirus disease COVID-19.

The use of a pharmaceutical composition, including Aprotex^{®}, Gordox^{®} and Aerus^{®}, with aprotinin as the active ingredient and excipients, said pharmaceutical composition being anti-SARS-CoV-2 virus drug Antiprovir to be used for the prevention and treatment of coronavirus disease COVID-19.

The drug Antiprovir for the prevention and treatment of coronavirus disease COVID-19, which is a pharmaceutical composition containing 0.1% wt. ÷ 0.2% wt. aprotinin, optionally 0.2 %wt. ÷ 1.0% wt. excipients, and the rest is water for injection.

## Description

This invention relates to a novel Anti-SARS-CoV-2 virus agent Antiprovir to be used for the prevention and treatment of COVID-19 coronaviral disease. A sudden outbreak of the new coronavirus (later named SARS-CoV-2) in Wuhan, China, in 2019, which quickly turned into a global pandemic, marked the third introduction of a virulent Coronavirus into human society, affecting not only the health system, but also the global economy. Effective approaches to vaccination, prevention, and treatment of SARS-CoV-2 (COVID-19) and epidemiological control are still lacking.

In this regard, an intensive search for vaccines and therapeutic agents for the prevention and treatment of SARS-CoV-2 (COVID-19) is underway worldwide. One practical approach as a rapid response to an emerging pandemic is to repurpose existing therapeutic agents previously intended for other viral infections, since most of these agents have already been tested for their safety. These agents can be divided into two broad categories: those that can directly target the virus replication cycle and those that are based on an immunotherapy approach aimed either at enhancing innate antiviral immune responses or at reducing the damage caused by dysregulation of inflammatory responses.

Initial clinical trials revealed a promising therapeutic potential for several such drugs, including favipiravir, a broad-spectrum antiviral drug that interferes with viral replication; hydroxychloroquine, a repurposed anti-malarial drug that interferes with the endosomal pathway of the virus; and remdesivir with a broad spectrum of antiviral activity. Remdesivir is an experimental drug that has no proven safety or efficacy for the treatment of any condition. Remdesivir has demonstrated activity in vitro and in vivo in animal models against the viral pathogens MERS and SARS, which are also coronaviruses and are structurally similar to COVID-19. Limited preclinical data on remdesivir in MERS and SARS indicate that remdesivir could be potentially active against COVID-19.

As of April 26, 2020, the COVID-19 coronavirus affected 210 countries and territories with a total of 3,055,308 cases of COVID-19 infected individuals, of which 917,432 people recovered and 211,032 people died.

Given that SARS-CoV-2 poses a serious threat to the world's public health and economy, it seems appropriate to search for novel potent anti-coronavirus drugs.

Listed below are definitions of various terms used throughout the specification of this invention.

The term **"medicinal drug"** refers to a compound (or a mixture of compounds forming a pharmaceutical composition) in tablets, capsules, injections, ointments, or other finished dosage forms intended for the restoration, improvement, or modification of physiological functions in humans and animals as well as for the treatment and prophylaxis of diseases, for diagnostics, anesthesia, contraception, cosmetology, etc.

The term **"pharmaceutical composition"** refers to a composition comprising a compound of formula 1 and at least one of the components selected from the group consisting of pharmaceutically acceptable and pharmacologically compatible fillers, solvents, diluents, carriers, excipients, distributing, and sensing agents, delivery agents such as preservatives, stabilizers, fillers, disintegrators, moisteners, emulsifiers, suspending agents, thickeners, sweeteners, flavoring agents, aromatizing agents, antibacterial agents, fungicides, lubricants, and prolonged delivery controllers, the choice and proportions of which depend on the nature and route of administration and dosage. Examples of suitable suspending agents are ethoxylated isostearyl alcohol, polyoxyethylene, sorbitol and sorbitol ether, microcrystalline cellulose, aluminum metahydroxide, bentonite, agar-agar and tragacant, and mixtures thereof. Protection against microorganisms can be provided using various antibacterial and antifungal agents, such as parabens, chlorobutanol, sorbic acid, and the like. Said composition may also include isotonic agents, such as sugar, sodium chloride, and the like. The sustained action of the composition can be achieved using agents that decelerate the absorption of the active ingredient, for example, aluminum monostearate and gelatin. Examples of suitable carriers, solvents, diluents and delivery agents include water, ethanol, polyalcohols and mixtures thereof, natural oils (such as olive oil), and organic esters (such as ethyl oleate) for injections. Examples of fillers are lactose, milk sugar, sodium citrate, calcium carbonate, calcium phosphate, and the like. Examples of disintegrators and distributors are starch, alginic acid and salts thereof, and silicates. Examples of lubricants are magnesium stearate, sodium lauryl sulfate, talc, and polyethylene glycol of high molecular weight. A pharmaceutical composition for oral, sublingual, transdermal, intramuscular, intravenous, subcutaneous, and local or rectal administration of the active ingredient, alone or in combination with another active ingredient, may be administered to animals and people in a standard administration form as a mixture with traditional pharmaceutical carriers. Suitable standard administration forms include oral forms, such as tablets, gelatin capsules, pills, powders, granules, chewing gums, and oral solutions or suspensions; sublingual and transbuccal administration forms; aerosols; implants; local, transdermal, subcutaneous, intramuscular, intravenous, intranasal, or intraocular forms; and rectal administration forms.

The term **"inert filler"** as used herein refers to a compound that is used for preparing a pharmaceutical composition and is, as a rule, safe, nontoxic, and neither biologically nor otherwise undesirable, and comprises excipients acceptable for veterinary and human pharmaceutical use. Compounds of this invention may be administered individually, but they will be generally administered in a mixture with one or more pharmaceutically acceptable excipients, diluents, or carriers chosen depending on the contemplated route of drug administration and standard pharmaceutical practice.

The term **"therapeutically effective amount,"** as used herein, refers to an amount of a substance, prodrug, or drug needed for alleviating the symptoms of the disease in the subject. The dose of a substance, prodrug, or drug will meet individual demands in each particular case. Said dose may vary in a wide range depending on numerous factors like the severity of the disease to be treated, the age and the general condition of the patient, other medicaments used for the patient's treatment, the mode and route of administration, and the experience of the attending doctor. For oral administration, the daily dose is approximately 0.01-10 g, including all values there between, both in monotherapy and/or combination therapy. The preferred daily dose is around 0.1-7 g. As a rule, in order to alleviate or eliminate the virus, a higher loading dose is given at the beginning of treatment with a subsequent reduction of the dose to a level sufficient to prevent an infection outburst.

The term **"subject"** refers to a mammal including, but not limited to, cattle, hogs, sheep, chickens, turkeys, buffalos, lamas, ostriches, dogs, cats, and humans; a human subject is most preferable. It is assumed that a subject's treatment may involve the use of any prodrug of general formula 1, its stereoisomer, isotopically enriched analog, pharmaceutically acceptable salt, hydrate, solvate, and crystalline or polymorphic form or their combinations with another compound, including with an inhibitor of RNA-dependent RNA polymerase (RdRp) of various RNA viruses.

Aprotinin, a polypeptide extracted from cattle organs, is known to have antiproteolytic, hemostatic, and antifibrinolytic effects and is the core component of a number of drugs for the treatment of various diseases such as Aprotex^{®}, Gordox^{®}, Aerus^{®}, etc.

Aprotex^{®} is a pharmaceutical composition comprising aprotinin lyophilizate as the active ingredient and excipients (lactose, sodium hydroxide) and is used for the preparation of a solution for intravenous administration [https://www.rlsnet.ru/tn_index_id_26457.htm] and has antiproteolytic, hemostatic, and antifibrinolytic effects. Aprotex is used for the treatment of acute or aggravated chronic pancreatitis and pancreonecrosis; bleeding on the background of hyperfibrinolysis: post-traumatic, postoperative (especially in operations on the prostate, lungs), before, after and during childbirth (including amniotic fluid embolism); polymenorrhea; angioedema; shock (toxic, traumatic, burn, hemorrhagic); extensive and deep traumatic tissue injuries, as well as for preventing acute non-specific postoperative mumps and performing diagnostic tests and operations on the pancreas (prevention of enzymatic autolysis during operations on the pancreas and adjacent abdominal organs) [https://www.vidal.ru/drugs/aprotex_30966].

Gordox^{®} is an injectable pharmaceutical composition comprising aprotinin as the active ingredient, excipients (sodium chloride, benzyl alcohol, and water for injection) and having an antifibrinolytic effect [https://yandex.ru/health/pills/product/gordoks-203]. Gordox^{®} s used to prevent intraoperative blood loss and reduce the volume of hemotransfusion during aortic bypass surgery using CPR in adult patients who are at increased risk of bleeding or in need of hemotransfusion [https://yandex.ru/health/pills/product/gordoks-203]. The drug is also recommended as a preventive treatment for patients who may have an increased risk of bleeding or need for transfusion [https://www.vidal.by/poisk_preparatov/ gordox.html].

Aerus^{®}-an aerosol inhaler [WO2012008869, RU2425691, EP2594283] for the treatment of viral respiratory infections and infectious and inflammatory diseases of viral etiology-is a pharmaceutical composition comprising aprotinin as the active ingredient and excipients (propellant: 1,1,1,2-tetrafluoroethane; solvents: ethanol, glycerol, and water; and stabilizer: peppermint oil) [https://www.vidal.ru/drugs/aerus_23575; https://www.rlsnet.ru/tn_index_id_44141.htm].

The inventors have surprisingly discovered that well-known pharmaceutical compositions, including Aprotex^{®} , Gordox^{®} and Aerus^{®}, which comprise aprotinin as the active ingredient and excipients, can be used in a new way, namely, as an anti-SARS-CoV-2 virus drug Antiprovir for the prevention and treatment of COVID-19 coronavirus disease.

The subject of this invention is the drug Antiprovir for the prevention and treatment of coronavirus disease COVID-19, which is a pharmaceutical composition containing 0.1% wt. ÷ 0.2% wt. aprotinin, optionally 0.2 %wt. ÷ 1.0% wt. excipients and the rest is water for injection. Possible excipients include sodium chloride, sodium hydroxide, benzyl alcohol, lactose, 1,1,1,2-tetrafluoroethane, peppermint leaf oil, ethanol, glycerol, etc.

Preferably, the Antiprovir drug is Gordox^{®}.

Preferably, the Antiprovir drug is Aerus^{®} .

Preferably, the Antiprovir drug is Aprotex^{®}.

The subject of this invention is a lyophilizate for the preparation of Antiprovir of this invention, which contains 1÷2 weight parts of aprotinin and optionally 2 ÷ 10 weight parts of excipients. Possible excipients include sodium chloride, sodium hydroxide, benzyl alcohol, lactose, 1,1,1,2-tetrafluoroethane, peppermint leaf oil, ethanol, glycerol, etc.

The subject of this invention is a method for the production of Antiprovir of this invention by dissolving the lyophilizate of this invention in water for injection.

Evaluation of the Covid-014 antiviral efficacy of Antiprovir of this invention in Vero E6 cell culture against the SARS-CoV-2 virus. Antiprovir was studied as a lyophilizate of the drugs Aprotex, Gordox and Aerus at a concentration of ~53,000 KIU/ml (or ~10,000 KIU+/well) against the SARS-CoV-2 virus by analyzing viral production by cytopathic action in Vero E6 cell culture with real-time PCR confirmation. It was found that Antiprovir of this invention, at a concentration of ~53,000 KIE/ml (or ~10,000 KIE / well), completely suppressed the cytopathic action of the virus at a dose of 100 TCID₅₀/well and partially suppressed the same at a dose of 1000 TCID₅₀/well.

Anti-SARS-CoV-2 viral efficacy of Antiprovir of this invention was evaluated using Polymerase Chain Reaction (PCR). The SARS-CoV-2 virus was detected in the studied wells in real time. The supernatant was collected from three dilution wells of the drug for 0 TCID₅₀, 100 TCID₅₀ and 1000 TCID₅₀ of the virus to be further used for RNA isolation in parallel with positive and negative control of the isolation stage. The isolated RNA was used for real-time PCR. The presence of viral RNA was evaluated by the threshold cycle value (Ct). PCR data showed complete inhibition of the SARS-CoV-2 virus by Antiprovir at a concentration of ~53,000 KIU/ml with a viral load of 100TCD₅₀.

Another subject of the present invention is a method for the prevention and treatment of SARS-CoV-2 (COVID-19) infections, said method involving administration to the patient of the drug Antiprovir of this invention.

Medicinal drugs can be administered parenterally, including intravenously, subcutaneously, abdominally, or via a spray device. The clinical dosage of the drug of this invention can be adjusted depending on the therapeutic effectiveness in the patient's body, their rate of metabolism and clearance as well as on the age, gender and phase of the disease. According to the instructions of the doctor or pharmacist, these medications may be taken several times within certain periods (preferably, from one to six times).

For the prevention of SARS-CoV-2 (COVID-19) in persons who have been in close contact with infected individuals and medical personnel, Antiprovir of this invention is taken twice a day at a dose of 300 KIU (units of activity) as inhalations or a nasal spray in each nostril for 14 days. For emergency prevention, Antiprovir is taken 4 times a day at a dose of 300 KIU as inhalations or a nasal spray in each nostril for 14 days.

For mild forms of COVID-19 infection, Antiprovir inhalation is taken 4 times a day for 7 days. In moderate cases, Antiprovir dosage can be increased to 5.0 KIU/patient/day with inhalations taken each 2 hours (600 KIU to 5000 KIU) for 7 days.

For severe forms of COVID-19, Antiprovir dosage is 30000 KIU/patient/day and can be increased up to 50,000 KIU/patient/day. This dosage will maintain the drug's therapeutic concentration in the lungs and other important organs at a level sufficient to suppress virus reproduction and reduce systemic inflammation. Antiprovir is administered slowly jet- or dropwise at a dose of 100,000 KIU 2-3 times a day for 5-7 days or 150,000 KIU 3 times a day for 5-7 days.

This invention is illustrated by, but not limited to, the following examples.
**Example 1.** Lyophilizate for the preparation of Antiprovir intended for COVID-19 intravenous or spray therapy. Aprotinin (1.5 g), sodium chloride (85 g), and benzyl alcohol (100 g) are dissolved in 10 1 of water for injection. The resulting solution is poured into suitable vials (10 ml per vial) using the Rota FLR 50 filling machine. Filled vials are sterilized at 120-122°C at a pressure of 120 kPa for 8 minutes, lyophilized in a Zirbus sublimator under a vacuum of less than 0.3 bar, corked, crimped with aluminum caps, and packed in 10-piece boxes to obtain lyophilizate for the preparation of Antiprovir, 100000 KIU in 10 ml.
**Example 2.** Anti-SARS-CoV-2 agent Antiprovir for intravenous therapy of COVID-19. Aprotinin (1.5 g), sodium chloride (85 g), and benzyl alcohol (100 g) are dissolved in 10 1 of water for injection. Half of the resulting solution is poured into suitable 1 ml ampoules of neutral glass and the other half, into 10 ml ampoules using the Rota FLR 50 filling machine. Filled ampoules are sterilized at 120-122°C at a pressure of 120 kPa for 8 minutes, sealed with a gas burner, and packed in 10-piece boxes to obtain an Antiprovir kit of 10000 KIU in 1 ml and 100000 KIU in 10 ml for COVID-19 intravenous therapy.
**Example 3.** Anti-SARS-CoV-2 medication Antiprovir for COVID-19 spray therapy. Aprotinin (1.5 g), sodium chloride (57 g), and benzyl alcohol (67 g) are dissolved in 101 of water for injection. The resulting solution is sterilized at 120-122°C at a pressure of 120 kPa for 8 minutes and dispensed into appropriate spray cans, 100 ml each (for nose and/or throat), to obtain anti-SARS-CoV-2 viral agent Antiprovir for COVID-19 spray therapy.
**Example 4.** Evaluation of the Covid-014 antiviral efficacy of Antiprovir in Vero E6 cell culture against SARS-CoV-2 virus. Antiprovir was studied as Aprotex and Gordox lyophilizates at a concentration of ~53 000 KIU/ml against the SARS-CoV-2 virus. Virus production was evaluated in terms of cytopathic effect in Vero E6 cell culture with real-time PCR confirmation at the Testing Center for Quality Control of Immunobiological Drugs, Gamaleya Federal Research Center for Epidemiology & Microbiology. Vero E6 cells were placed in 96-well culture flat-bottomed plates (12000 cells/well) in 100.0 µl of freshly prepared complete culture medium (CM). The cells were cultured for 24 h at 37°C, 5% CO₂. To prepare a two-fold dilution of the test drug at a concentration of 106,400 U/ml, the contents of 8 vials was serially dissolved in 1 ml of the reaction medium (RM). The resulting 106,400 KIU/ml solution was kept for 2 hours at 2-8°C to control the drug's solubility. CM was removed from the plates, and the cells were washed with RM medium and filled with 100 µl of each dilution of test substance. Each dilution point was tested in 3 wells (in triplicate). In addition, the drug was added to virus-free control wells in order to evaluate potential cytotoxic effects and keep a research record. Pure RM was added to the cell control wells. To infect cells, a suspension of SARS-CoV-2 virus (passage 4) with an infection activity of 10⁶ TCID₅₀/1 ml for Vero E6 cells was prepared. A series of 10-fold virus dilutions were made: 10⁻¹ and up to 10⁻⁶. The suspension was diluted by successive transfer to test tubes with the necessary amount of corresponding RM: 900 µl of RM and 100 µl of virus suspension in each tube. To prepare a virus suspension at a concentration of 1000 TCID₅₀/ml, 10 µl of the suspension was taken from the stock virus suspension at a concentration of 1×10⁶TCID₅₀/ml and placed in 10.0 ml of RM. To prepare a virus suspension at a concentration of 10000 TCID₅₀/ml, 100 µl of the suspension was taken from the stock virus suspension at a concentration of 1×10⁶TCID₅₀/ml and placed in 9.9 ml of RM. The dilutions of the virus suspension were added to the cells following their 2-hour incubation with the dilution of the tested drug and then co-incubated them for 96 hours. The virus (100 µl) at concentrations of 1000 and 10000 TCID₅₀ /ml (100 and 1000 TCID₅₀ per well, respectively) was added to wells 100 TCID₅₀ and 1000 TCID₅₀ in RM medium. RM (100 µl) was added to each cell control well. Plates with cells were incubated for 96 hours at 37°C, 5% CO₂ until the virus in the viral control fully manifested the cytopathic effect in the expected range. The antiviral activity of the samples was evaluated visually under a microscope 96 hours after infection by the inhibition of the Cytopathic Effect (CPE) of the virus in Vero E6 cell culture. The study culminated in a report on the inhibition of the cytopathic effect of the virus in Vero E6 cell culture upon exposure to the drug: complete inhibition (CPE absence in 3 wells out of 3), incomplete inhibition (CPE presence in 1 or 2 wells out of 3), absence of inhibition (CPE presence in 3 wells out of 3). Outcome: Antiprovir at a concentration of ~53 000 KIU/ml (or ~10 000KIU/well) completely suppressed the cytopathic effect of the virus at a dose of 100 TCID₅₀/well and partially suppressed said effect at a dose of 1000 TCID₅₀/well.

The efficacy of anti-SARS-CoV-2 virus action of the drug in the studied wells was determined in real time using the polymerase chain reaction (PCR) method. From three wells of drug dilution for 0 TCID₅₀, 100 TCID₅₀ and 1000 TCID₅₀ of the virus, supernatant was removed and used for RNA isolation in parallel with positive and negative control of the isolation stage. The isolated RNA was used for real-time PCR. The presence of viral RNA was evaluated in terms of threshold cycle (Ct). The outcome is as follows: PCR data showed complete inhibition of SARS-CoV-2 virus reproduction at Antiprovir concentration of ~53,000 KIU/ml and confirmed high anti-SARS-CoV-2 virus activity of Antiprovir.

## Claims

1. The use of a pharmaceutical composition, including Aprotex^{®}, Gordox^{®} and Aerus^{®}, containing aprotinin as the active ingredient and excipients, said composition being anti-SARS-CoV-2 virus drug Antiprovir intended for the prevention and treatment of coronavirus disease COVID-19.

2. The drug Antiprovir for the prevention and treatment of coronavirus disease COVID-19, which is a pharmaceutical composition containing 0.1% wt. ÷ 0.2% wt. aprotinin, optionally 0.2 %wt. ÷ 1.0% wt. excipients, and the rest is water for injection.

3. Antiprovir according to Claim 2 containing excipients selected from sodium chloride, sodium hydroxide, benzyl alcohol, lactose, 1,1,1,2-tetrafluoroethane, peppermint leaf oil, ethanol, and glycerol.

4. Antiprovir according to Claim 2, which is the drug Gordox^{®}.

5. Antiprovir according to Claim 2, which is the drug Aprotex^{®}.

6. Antiprovir according to Claim 2, which is the drug Aerus^{®}.

7. A lyophilizate for the preparation of the drug Antiprovir containing 0.1% wt. ÷ 0.2% wt. aprotinin and optionally 0.2 %wt. ÷ 1.0% wt. excipients.

8. The lyophilizate according to Claim 7 containing excipients selected from sodium chloride, sodium hydroxide, and lactose.

9. A method for the production of Antiprovir of this invention by dissolving the lyophilizate according to Claims 7, 8 in water for injection

10. A method for the prevention and treatment of SARS-CoV-2 (COVID-19) infections involving the administration to the patient of the drug Antiprovir of this invention.

11. The method according to Claim 10 involving intravenous therapy.

12. The method according to Claim10 involving spray therapy.

13. The method according to Claims 10-12 involving the administration to the patient of the drug Antiprovir at a dose of 300-150,000 KIU from 2 to 4 times a day for 5- 14 days.
